(19) Europäisches Patentamt
European Patent Office
Office européen des brevets

(11) **EP 3 629 335 A1**

(12) **EUROPÄISCHE PATENTANMELDUNG**

(43) Veröffentlichungstag:
**01.04.2020 Patentblatt 2020/14**

(51) Int Cl.:
***G16H 30/20*** *(2018.01)*

(21) Anmeldenummer: **18196783.7**

(22) Anmeldetag: **26.09.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH
91052 Erlangen (DE)**

(72) Erfinder: **Hammes, Martin
91054 Erlangen (DE)**

(54) **VERFAHREN ZUM AUTOMATISCHEN AUSWÄHLEN VON BILDGEBUNGSPARAMETERN FÜR BILDGEBUNGSVERFAHREN**

(57) Die vorliegende Erfindung betrifft ein automatisches Verfahren zum Auswählen von Bildgebungsparametern für Bildgebungsverfahren, ein entsprechendes Bildgebungsverfahren und eine entsprechende Bildgebungsvorrichtung umfassend einen Erkennungsalgorithmus (Detection Algorithm) und einen Auswahlalgorithmus (Selection Algorithm). Es erfolgt ein Erstellen einer Rangliste mindestens eines qualifizierenden Werts für potentielle klinische Indikationen. Die Rangliste basiert auf Bilddaten. Das Erstellen der Rangliste erfolgt mittels des Erkennungsalgorithmus. Zudem erfolgt ein Auswählen wenigstens eines Bildgebungsparameters. Der wenigstens eine Bildgebungsparameter ist geeignet, Bilddaten zu erzeugen, die eine maximale Änderung der qualifizierenden Werte in der Rangliste ermöglichen. Das Auswählen des wenigstens einen Bildgebungsparameters erfolgt mittels des Auswahlalgorithmus.

FIG 1

**Beschreibung**

Die vorliegende Erfindung betrifft ein automatisches Verfahren zum Auswählen von Bildgebungsparametern für Bildgebungsverfahren, ins besondere zum Auswählen von Magnetresonanzto-mographie-(MRT-)Sequenzen, ein entsprechendes Bildgebungsverfahren, insbesondere ein MRT-Verfahren, und eine entsprechende Bildgebungsvorrichtung, insbesondere ein MRT-Gerät, umfassend einen Erkennungsalgorithmus (Detection Algorithm) und einen Auswahlalgorithmus (Selection Algorithm).

Bei der Erstellung von medizinischen Bilddaten von Patienten, insbesondere mit einem MRT- Gerät bzw. Magnetresonanz-(MR-)Scanner, aber auch mit einem Computertomographie-(CT-)Gerät oder vergleichbaren Bildgebungsgeräten, werden üblicherweise mehrere Bilddaten mit verschiedenen Bildgebungsparametern wie Sequenzen bzw. Kontraste mit unterschiedlichen Orientierungen, Schichtdicken usw. aufgenommen. Dabei werden für unterschiedliche klinische Fragestellungen typischerweise unterschiedliche Bildgebungsparameter verwendet. Bildgebungsvorrichtungen (z. B. MRT-Geräte) können typischerweise so konfiguriert werden, dass eine Kombination aus verschiedenen Bildgebungsparametern als Protokoll gespeichert wird. Diese Protokolle können dann herangezogen werden, um Patienten mit bestimmten klinischen Fragestellungen zu scannen, um möglichst optimale Bilddaten zur Diagnosestellung zu erhalten. Bei Patienten mit einer klaren Überweisung bzw. klinischen Fragestellung, beispielsweise "Follow-Up für Multiple Sklerose", wird üblicherweise ein "Multiple-Sklerose-Protokoll" ausgewählt und damit optimale Bilddaten zur Befundung der klinischen Fragestellung erzeugt werden.

Im Alltag tritt allerdings öfters das Problem auf, dass Patienten mit unklaren Befunden überwiesen werden bzw. in der Radiologie ankommen. Oft sind auch die klinischen Fragestellungen unklar (beispielsweise "unklarer Kopfschmerz"). In diesen Fällen werden oftmals Standard-Protokolle herangezogen und nach der Aufnahme mit bestimmten Bildgebungsparametern (z. B. Sequenzen/Kontrasten) prüft ein medizinischer Fachangestellter (Medizinisch-Technischer RadiologieAssistent, MTRA) die entsprechenden Bilddaten bzw. Bilder (teilweise mit Unterstützung von Radiologen). Wenn dann ein Verdacht auf eine bestimmte klinische Indikation besteht, wird das StandardProtokoll angepasst, bevor die Bildgebung fortgesetzt wird, damit am Ende der Bildgebung der Radiologe die Bilder vorliegen hat, die er für eine möglichst optimale Befundung des Verdachtsfalls benötigt. Das heißt es werden besondere Bildgebungsparameter (z. B. Kontraste oder besondere Orientierungen, Schichtdicken usw.) eingestellt und die Bildgebung wird mit einem entsprechend angepassten Protokoll fortgesetzt.

Dieses Vorgehen hat jedoch mitunter gravierende Nachteile. Zum einen kann die Befundung der Bilder zeitaufwändig sein, insbesondere dann, wenn erst Radiologen hinzugerufen werden müssen. Dieser Zeitaufwand kann zu Verzögerungen im Ablauf führen. Dies ist unangenehm für den Patienten (z. B. Wartezeit in engem MR-Scanner) und kann gegebenenfalls bei einer zeitkritischen Diagnose (z. B. Schlaganfall) sogar zu einer schwerwiegenden Schädigung des Patienten führen. Zum anderen muss der MTRA sehr gut ausgebildet sein, um geeignete Bildgebungsparameter basierend auf den Bildern mit dem StandardProtokoll auswählen zu können. Wenn der MTRA schlecht ausgebildet ist, kann es somit sein, dass das Standardprotokoll durchlaufen wird und erst im Nachhinein bei der Befundung durch den Radiologen auffällt, dass zusätzliche Bilder bzw. Bilddaten mit angepassten Bildgebungsparametern benötigt werden. In diesem Fall muss der Patient neu einbestellt werden, was zusätzlichen Zeitaufwand und Kosten verursacht. Hinzukommt, dass nicht immer ein Radiologe sofort zum direkten Betrachten der Bilder (insbesondere im Fall schlecht ausgebildeter MTRA) verfügbar ist. Es wird auch eine enorme Kapazität der Radiologen durch diese "live" Betrachtung der Bilder gebunden. Wenn unnötige Bilddaten aufgenommen werden und dann später noch mehr Bilddaten mit entsprechend angepassten Bildgebungsparametern hinzugefügt werden müssen, die für die eigentliche Diagnose benötigt werden, so wird zusätzlich wertvolle Zeit verschwendet. Überdies müssen die Radiologen auch "unnötige" Bilder befunden, wodurch noch mehr Kapazität verschwendet wird.

Heutzutage wird dieses Problem durch den Einsatz von qualifiziertem Personal gelöst (MTRA + Radiologe) oder dadurch, dass in das Standardprotokoll die Bildgebungsparameter (z. B. Kontraste und Sequenzen) für alle potentiellen klinischen Fragestellungen aufgenommen werden. Letzteres Vorgehen führt allerdings dazu, dass die Untersuchungsdauer sehr lange ist, was sehr unangenehm für den Patienten sein kann , nicht wirtschaftlich ist und schlimmsten Falls sogar zur Schädigung des Patienten führen kann (z. B. durch Zeitverlust im Falle eines Schlaganfalls).

Die vorliegende Erfindung hat daher die Aufgabe, ein Verfahren bereitzustellen, mit dem so zeitsparend wie möglich möglichst optimale Bildgebungsparameter zur Erstellung von Bilddaten bzw. Bildern für die Befundung ermittelt werden.

Zur Lösung der genannten Aufgabe stellt die vorliegende Erfindung ein Verfahren zum automatischen Auswählen von Bildgebungsparametern gemäß Anspruch 1 sowie ein entsprechendes Bildgebungsverfahren und eine Bildgebungsvorrichtung gemäß der weiteren unabhängigen Ansprüche bereit. Vorteilhafte Ausgestaltungen und Weiterbildungen der vorliegenden Erfindung sind Gegenstand der jeweils abhängigen Ansprüche. Einzelne Merkmale und Merkmalskombinationen der Ausgestaltungen und Weiterbildungen können, außer dies wird nachfolgend explizit ausgeschlossen, beliebig in technologisch sinnvoller Weise miteinander kombiniert werden. Die so gebildeten weiteren Ausführungsformen und Weiterbildung sind für den Fachmann ersichtlich von der vorliegenden Erfindung ebenfalls umfasst und gehören daher mit in deren Schutzbereich.

Gemäß einem ersten Aspekt der vorliegenden Erfindung umfasst ein Verfahren zum automatischen Auswählen von Bildgebungsparametern die folgenden iterativen (sequenziellen) Schritte:

a) Empfangen von Bilddaten, die von einem Bildgebungsmodul stammen können, beispielsweise durch ein Auswahlmodul.

b) Erstellen einer Rangliste mindestens eines qualifizierenden Werts für potentielle klinische Indikationen. Die Rangliste basiert auf zumindest den Bilddaten aus dem vorherigen Schritt. Das Erstellen der Rangliste erfolgt mittels eines Erkennungsalgorithmus (Detection Algorithm, DA), beispielsweise durch das Auswahlmodul.

c) Auswählen wenigstens eines Bildgebungsparameters. Der wenigstens eine Bildgebungsparameter ist geeignet, Bilddaten zu erzeugen, die eine maximale Änderung der qualifizierenden Werte in der Rangliste ermöglichen. Das Auswählen des wenigstens einen Bildgebungsparameters erfolgt mittels eines Auswahlalgorithmus (Selection Algorithm, SA), beispielsweise durch das Auswahlmodul.

d) Senden des ausgewählten Bildgebungsparameters, beispielsweise an das Bildgebungsmodul, beispielsweise durch das Auswahlmodul.

Die Schritte a) bis d) werden wiederholt, bis ein vordefiniertes Abbruchkriterium erfüllt ist.

Gemäß einem zweiten Aspekt der vorliegenden Erfindung schließt ein Bildgebungsverfahren das Verfahren zum automatischen Auswählen von Bildgebungsparametern wie zuvor beschrieben ein. Das Bildgebungsverfahren umfasst die folgenden iterativen (sequentiellen) Schritte:

A) Laden wenigstens eines primären Bildgebungsparameters, beispielsweise durch ein Bildgebungsmodul.

B) Durchführen einer Bildgebung basierend auf dem geladenen Bildgebungsparameter und Generieren von Bilddaten, beispielsweise mit der Bildgebungsvorrichtung, beispielsweise durch das Bildgebungsmodul.

C) Senden der generierten Bilddaten, beispielsweise an ein Auswahlmodul, beispielsweise durch das Bildgebungsmodul.

a) Empfangen der Bilddaten, die von dem Bildgebungsmodul stammen können, beispielsweise durch das Auswahlmodul.

b) Erstellen einer Rangliste mindestens eines qualifizierenden Werts für potentielle klinische Indikationen. Die Rangliste basiert auf zumindest den Bilddaten aus dem vorherigen Schritt. Das Erstellen der Rangliste erfolgt mittels eines Erkennungsalgorithmus (Detection Algorithm, DA), beispielsweise durch das Auswahlmodul.

c) Auswählen wenigstens eines Bildgebungsparameters. Der wenigstens eine Bildgebungsparameter ist geeignet, Bilddaten zu erzeugen, die eine maximale Änderung der qualifizierenden Werte in der Rangliste ermöglichen. Das Auswählen des wenigstens einen Bildgebungsparameters erfolgt mittels eines Auswahlalgorithmus (Selection Algorithm, SA), beispielsweise durch das Auswahlmodul.

d) Senden des ausgewählten Bildgebungsparameters, beispielsweise an das Bildgebungsmodul, beispielsweise durch das Auswahlmodul.

D) Empfangen des ausgewählten Bildgebungsparameters, der von dem Auswahlmodul stammen kann, beispielsweise durch das Bildgebungsmodul.

E) Laden des empfangenen Bildgebungsparameters, beispielsweise durch das Bildgebungsmodul.

Die Schritte B) bis C), a) bis d) und D) bis E) werden wiederholt, bis ein vordefiniertes Abbruchkriterium erfüllt ist.

Gemäß einem dritten Aspekt der vorliegenden Erfindung umfasst eine Bildgebungsvorrichtung eine Steuereinrichtung mit einem Bildgebungsmodul und einem Auswahlmodul. Die Steuereinrichtung ist eingerichtet, das zuvor beschriebene Bildgebungsverfahren durchzuführen. Das Bildgebungsmodul ist eingerichtet, die Schritte A) bis C) und D) bis E) auszuführen. Das Auswahlmodul ist eingerichtet, die Schritte a) bis d) auszuführen.

Kern der vorliegenden Erfindung ist, dass eine Kombination aus dem Erkennungsalgorithmus (DA) und dem Auswahlalgorithmus (SA) aufgenommene Bilddaten analysiert und das Ergebnis der Bildanalyse direkten Einfluss auf das darauffolgende Verhalten des Bildgebungsverfahrens bzw. der Bildgebungsvorrichtung (MR-Scanner) hat, indem Bildgebungsparameter (z. B. Kontraste oder Sequenzen) basierend auf dem besagten Ergebnis der Bildanalyse automatisch ausgewählt werden.

Wenn eine neue Bildgebung gestartet wird, insbesondere für einen Patienten mit unklarem Befund bzw. unklarer klinischer Fragestellung, wird zunächst wenigstens ein primärer Bildgebungsparameter von dem Bildgebungsmodul geladen. Dieser wenigstens eine primäre Bildgebungsparameter kann ein Standardprotokoll bzw. ein Primärprotokoll definieren oder von diesem umfasst sein. Das Standardprotokoll kann verwendet werden, um beispielsweise eine Übersichtsaufnahme des fraglichen Körperteils oder sogar des gesamten Patienten (spiral-CT-Aufnahme) zu generieren. Mit dem wenigstens einen geladenen primären Bildgebungsparameter werden (primäre) Bilddaten erzeugt, die als Grundlage für die nachfolgende Auswahl von Bildgebungsparametern dienen. Die generierten (primären) Bilddaten werden dazu von dem Bildgebungsmodul an das Auswahlmodul übermittelt.

Nachdem das Auswahlmodul die (primären) Bilddaten empfangen hat, wird basierend auf den Bilddaten von dem Erkennungsalgorithmus (DA) eine Rangliste mit wahrscheinlichen klinischen Indikationen bzw. Diagnosen erstellt. Die Rangliste basiert auf wenigsten einem qualifizierenden Wert für die klinischen Indikationen (z. B. deren Wahrscheinlichkeit und/oder eine Konfidenz/Signifikanz dieser Wahrscheinlichkeit). Dabei führt der Erkennungsalgorithmus (DA) eine Bildanalyse der Bilddaten durch. Der Auswahlalgorithmus kann dazu Künstliche-Intelligenz-(KI-)basiert sein und beispielsweise ein Neuronales Netz (NN) bzw. ein tiefes NN (Deep NN, DNN) oder ein "Convolutional NN" (CNN) umfassen oder alternativ eine "Support Vector Machine" (SVM) oder einen "Random Forest Classifier" (RFC) umfassen. Ein Erkennungsalgorithmus basierend auf einem NN kann zunächst mit Trainingsdaten auf das Erkennen von klinischen Indikationen/Diagnosen oder auch nur einer bestimmten klinischen Indikation/Diagnose in Bilddaten trainiert werden. Dazu können Trainings-Bilddatensätze einzelner Bildgebungsparameter (z. B. Sequenzen/Kontraste) inklusive technischer Konfigurationsparameter, die beispielsweise in Metadaten wie einem DICOM Header gespeichert sein können, verwendet werden. Basierend auf einer Information ("Ground Truth") darüber, ob eine bestimmte klinische Indikation in den Bilddaten zu erkennen ist (z. B. als boolesche Variable: 1 = klinische Indikation vorhanden; 0 = klinische Indikation nicht vorhanden) wird das NN mit den besagten Trainings-Bilddatensätzen trainiert. Es können auch weitere Informationen beispielsweise darüber, welche Bilder zum gleichen Patienten gehören, in das Training des NN mit einfließen. Allgemein können zum Training und zum Test bzw. der endgültigen Validierung des NN unterschiedliche Strategien verwendet werden. Das trainierte NN kann als Erkennungsalgorithmus (DA) eingesetzt werden. Das NN kann auf die Bilddaten (beispielsweise im DICOM-Format) angewendet werden und für jede klinische Indikation/Diagnose, auf die das NN trainiert wurde, wenigstens einen entsprechenden qualifizierenden Wert ausgeben.

Basierend auf der Rangliste mit den qualifizierenden Werten zu den einzelnen klinischen Indikationen/Diagnosen wird anschließend der Auswahlalgorithmus (SA) ausgeführt. Ziel des Auswahlalgorithmus ist es, den wenigstens einen Bildgebungsparameter für die nächste Bildgebung bzw. Scansequenz auszuwählen. Der wenigstens eine Bildgebungsparameter wird dabei derart ausgewählt bzw. festgelegt, dass sich der wenigstens eine qualifizierende Wert für die jeweiligen klinischen Indikationen in der Rangliste in den nachfolgend aufgenommenen Bilddaten maximal verändern werden wird. Diese maximale Veränderung bedeutet beispielsweise, dass sich eine Wahrscheinlichkeit für eine klinische Indikation oder Diagnose (z. B. Schlaganfall) derart ändert, dass die klinische Indikation/Diagnose (sicher) ausgeschlossen (z. B. Wahrscheinlichkeit einer klinischen Indikation/Diagnose $\leq$ 5 % [Prozent]) oder (sicher) bestätigt (z. B. $P_{Indication} \geq 95$ %) werden kann. Sobald das Abbruchkriterium erfüllt ist, werden keine Bilddaten mehr empfangen und durch den Erkennungsalgorithmus analysiert bzw. die Rangliste erstellt. Es wird auch kein weiterer Bildgebungsparameter ausgewählt und an das Bildgebungsmodul gesendet. Das Abbruchkriterium ($T_{low}$, $T_{high}$) kann beispielsweise ein unterer und/oder oberer Grenzwert für die Wahrscheinlichkeit einer klinischen Indikation/Diagnose sein (z. B. 0,05 $\geq$ || 0,95 $\leq P_{Indication}$).

Der durch den Auswahlalgorithmus ausgewählte wenigstens eine Bildgebungsparameter wird von dem Auswahlmodul an das Bildgebungsmodul übertragen. Nachdem der wenigstens eine Bildgebungsparameter von dem Bildgebungsmodul empfangen wurde, wird dieser von dem Bildgebungsmodul für eine erneute Bildgebung geladen. Daraufhin wird die erneute Bildgebung mit dem entsprechenden wenigstens einen Bildgebungsparameter durchgeführt und die erhaltenen Bilddaten wieder dem Auswahlmodul zugeführt.

Sobald das bereits beschriebene Abbruchkriterium erfüllt ist, erfolgt auch keine weitere Bildgebung. Die letzten erstellten Bilddaten können, nach dem das Abbruchkriterium erfüllt ist, an einen Benutzer (MTRA und/oder Arzt bzw. Radiologe) ausgegeben werden. Optional können auch die letzte aktualisierte Rangliste und/oder die vorherigen Bilddaten mit ausgegeben werden. Der Benutzer kann dann eine Diagnose für den Patienten anhand der letzten erstellten Bilddaten und optional auch anhand der Rangliste und/oder den vorherigen Bilddaten stellen.

Optional können auch Informationen des überweisenden Arztes (z. B. klinische Fragestellung die abgeklärt werden soll (Verdacht auf eine bestimmte klinische Indikation/Diagnose)) bei dem Auswählen des wenigstens einen Bildgebungsparameters berücksichtigt werden.

Die Kosten für den Benutzer sinken aufgrund der Verringerung der Anzahl bzw. der Vermeidung von unnötigen "Re-Scans" und aufgrund des geringeren Personalaufwands. Patienten profitieren von dieser technischen Lösung, da sie kürzere "Scan-Times" haben, weil schneller festgestellt wird, welcher (mögliche) Befund vorliegt, und da sie eine bessere bzw. vollständigere Diagnose erhalten. Zudem kann auch ohne bzw. mit unvollständigen Informationen des überweisenden Arztes eine schnelle Generierung von Bilddaten erfolgen, die eine Abklärung (Bestätigung oder Ausschluss) der (zuvor unbekannten) wahrscheinlichsten klinischen Indikation oder Diagnose des Patienten ermöglichen, da das dynamische Protokoll aus Erkennungsalgorithmus (DA) und Auswahlalgorithmus (SA) die Informationen des überweisenden Arztes nur noch optional benötigt.

Gemäß einer Ausgestaltung der vorliegenden Erfindung erfolgt in Schritt c) das Auswählen des wenigstens einen Bildgebungsparameters durch den Auswahlalgorithmus (SA) anhand einer Datentabelle und/oder anhand eines Modells.

Es ist das Ziel des Auswahlalgorithmus, die nächste Bildgebung durch Auswählen wenigstens eines möglichst optimalen Bildgebungsparameters (z. B. Sequenz bzw. Kontrast) für das Bildgebungsverfahren bzw. die Bildgebungsvorrichtung (z. B. MR-Scanner) optimal zu gestalten. Basis für das Auswählen ist dabei das Ergebnis des vorhergehenden Erkennungsalgorithmus (DA) (insbesondere die Wahrscheinlichkeit für das Vorhandensein einer/mehrerer bestimmten/r klinischen/r Indikation(en)/Diagnose(n) und/oder die Konfidenz(en) dieser Wahrscheinlichkeit (en)).

Das Auswählen des wenigstens einen Bildgebungsparameters kann basierend auf der Datentabelle "memorybased" erfolgen, wobei die Informationen zu den Bildgebungsparametern (= Auswahloptionen, z. B. Sequenzen und weitere Einstellungen) und deren Eigenschaften in der Datentabelle vorliegen. Die Tabelle kann je Bildgebungsparameter beispielsweise drei Bereiche enthalten:

1. Bildgebungsparameter (z. B. Scansequenz und Sequenzeinstellungen (z. B. FLAIR, "Slice Thickness" usw.)).
2. Bildgebungseigenschaften (z. B. Zeitdauer, "Atemanhalten [ja/nein]" usw.)
3. Informationsgehalt für klinische Indikationen (z. B. boole'sch [ja = hat Informationswert; nein = hat keinen Informationswert] oder kontinuierlich [0 % = kein Informationswert bis 100% = Krankheit kann alleine auf Basis dieses Bildgebungsparameters bzw. dieses Protokolls erkannt werden]).

Auf diese Datentabelle können unterschiedliche Algorithmen zur Auswahl angewendet werden. Es kann beispielsweise zuerst ein Durchsuchen der Bildgebungsparameter in der Datentabelle und ein Auswählen der Option (Bildgebungsparameter und Bildgebungseigenschaften) für die die entsprechenden Suchkriterien gelten erfolgen. Anschließend kann ein Übergeben des ausgewählten wenigstens einen Bildgebungsparameters an das Bildgebungsverfahren bzw. die Bildgebungsvorrichtung zur Konfiguration erfolgen, sodass nun auf Basis der ausgewählten Option die erneute Bildgebung initiiert werden kann.

Das Auswählen des wenigstens einen Bildgebungsparameters kann auch basierend auf dem Modell "modelbased" erfolgen. Die Informationen zum präferierten wenigstens einen Bildgebungsparameter sind als Gewichte innerhalb eines mathematischen Modells (z. B. eines NN) gespeichert. Es kann ein Training eines NN auf Basis des Nutzerverhaltens und/oder der Nutzungshäufigkeit erfolgen:

1. Training des NN (inklusive Test und Validierung) mit klinischen Indikationen und dafür typischen Bildgebungsparametern (Sequenzen und Sequenzeinstellungen zur Diagnose bzw. Protokolle) als Trainingsdatensatz.
2. Nutzung des trainierten NN zum Auswählen des nächsten wenigstens einen Bildgebungsparameters (z. B. Scansequenz):

   a. Auswählen des Bildgebungsparameters (Scansequenz), der von den meisten Benutzern zur Diagnose einer spezifischen klinischen Indikation/Diagnose verwendet wurde (z. B. max(Häufigkeit klinische Indikation: Tumor))
   b. Auswählen des Bildgebungsparameters (z. B. Scansequenz), die von den meisten Nutzern im Durchschnitt zur Diagnose aller klinischen Indikation/Diagnose verwendet wurde (z. B. max(Durchschnitt[Häufigkeit alle klinische Indikationen]))

Die Datentabelle und das Modell können auch miteinander kombiniert werden.

Es besteht außerdem die Möglichkeit zwischen dem Erkennungsalgorithmus (DA) und dem Auswahlalgorithmus (SA) eine Rückführungsschleife ("feedback loop") einzufügen, sodass nach der Auswahl der nächsten Bildgebung und der Bestimmung der neuen Rangliste durch den Erkennungsalgorithmus (DA) diese Information an den Auswahlalgorithmus (SA) zurückgegeben wird und diese somit verwendet werden kann, um z. B. die Informationen in der Datentabelle zu verbessern oder das Modell bzw. die Gewichte im NN des Auswahlalgorithmus (SA) anzupassen.

Das Auswählen der Bildgebungsparameter durch den Auswahlalgorithmus (SA) anhand einer Datentabelle kann besonders einfach implementiert werden und ist daher kostengünstig. Werden stattdessen oder zusätzlich anhand eines Modells (z. B. NN) die Bildgebungsparameter ausgewählt, steht auch bei einer Vielzahl von Auswahlmöglichkeiten, die sich untereinander stark ähneln, ein sicheres Auswahlmittel zur Verfügung.

Gemäß einer Ausgestaltung der vorliegenden Erfindung ist das Abbruchkriterium erfüllt, wenn ein qualifizierender Wert wenigstens einer der klinischen Indikationen in der Rangliste ein vorgegebenes Grenzwertkriterium (z. B. Schwellwert für Konfidenz (Schwell-Konfidenz)) erfüllt oder alle möglichen Bildgebungsparameter durchlaufen sind.

Insbesondere kann, wenn eine oder mehrere klinische Indikation/Diagnose mit hoher Wahrscheinlichkeit vorliegt, die weitere Auswahl von Bildgebungsparametern und erneute Bildgebung abgebrochen werden. Zusätzlich oder alternativ kann auch, wenn eine oder mehrere klinische Indikationen/Diagnosen mit hoher Wahrscheinlichkeit ausgeschlossen werden können, die weitere Auswahl von Bildgebungsparametern und erneute Bildgebung abgebrochen werden. Es kann das Verfahren zum Auswählen bzw. das Bildgebungsverfahren abgebrochen werden, wenn der wenigstens eine qualifizierende Wert (z. B. Wahrscheinlichkeit einer klinischen Indikation/Diagnose $P_{Indication}$ und/oder deren Konfidenz $C_{Indication}$) wenigstens einer klinischen Indikation/Diagnose in der Rangliste das Abbruchkriterium $T_{low}$ ≤ 10 %, bevorzugt $T_{low}$ ≤ 5 %, besonders bevorzugt $T_{low}$ ≤ 2 %, am meisten bevorzugt $T_{low}$ ≤ 1 % und/oder $T_{high}$ ≥ 90 %, bevorzugt $T_{high}$ ≥ 95 %, besonders bevorzugt $T_{high}$ ≥ 98 %, am meisten bevorzugt $T_{high}$ ≥ 99 % erfüllt. es können für verschiedene qualifizierende Werte verschiedenen Abbruchkriterien ($T^{Indication}$, $T^{Confidence}$) vorgegeben werden.

$$(P_{Indication} \leq T_{low}^{Indication} \vee P_{Indication} \geq T_{high}^{Indication}) \vee (C_{Indication} \geq T^{Confidence})$$

Alternativ kann das Verfahren zum automatischen Auswählen bzw. das Bildgebungsverfahren abgebrochen werden, wenn alle möglichen Bildgebungsparameter, die für die klinischen Indikationen in der Rangliste in Frage kommen, durchlaufen sind.

Je nachdem welche klinische Indikation bzw. Diagnose abgeklärt werden muss kann ein restriktives oder weniger restriktives Abbruchkriterium voreingestellt werden. Dies trägt zur Optimierung der Anzahl an Iterationen des automatischen Auswählens von Bildgebungsparametern bei. Somit kann die benötigte Zeit für das Verfahren zum automatischen Auswählen bzw. für das Bildgebungsverfahren reduziert werden. Der Benutzer (z. B. Radiologe oder MTRA) hat somit die Möglichkeit über die Abbruchkriterien (z. B. Schwell-Konfidenz) das Verhalten der Bildgebungsvorrichtung bzw. des Bildgebungsverfahrens zu steuern.

Gemäß einer Ausgestaltung der vorliegenden Erfindung beruht in Schritt c) die maximale Änderung der qualifizierenden Werte in der Rangliste auf einer maximalen Änderung eines der qualifizierenden Werte einer einzelnen klinischen Indikation in der Rangliste oder auf einer kumulierten maximalen Änderung qualifizierender Werte mehrerer klinischer Indikationen in der Rangliste.

Sofern eine bestimmte klinische Indikation bzw. Diagnose abgeklärt (mit hoher Sicherheit bestätigt oder ausgeschlossen) werden soll, kann der wenigstens eine Bildgebungsparameter durch den Auswahlalgorithmus (SA) so ausgewählt werden, dass sich der wenigstens eine qualifizierende Wert nur für die abzuklärende klinische Indikation/Diagnose maximal ändert, sodass nach einer erneuten Bildgebung mit dem entsprechend ausgewählten wenigstens einen Bildgebungsparameter die abzuklärende klinische Indikation/Diagnose anhand der neuen Bilddaten so viel besser wie möglich bestätigt oder ausgeschlossen werden kann. Alternativ kann auch der wenigstens eine qualifizierende Wert für mehrere oder so viele klinische Indikationen/Diagnosen aus der Rangliste wie möglich so stark wie möglich verändert werden. Dazu werden die Änderungen in dem wenigstens einen qualifizierenden Wert zu der kumulierten Änderung (betragsmäßig) aufsummiert und dementsprechend dann die kumulierte maximale Änderung bestimmt.

Beispielsweise kann das Auswählen durch den Auswahlalgorithmus (SA) anhand folgender Kriterien erfolgen:

a. Fokus auf breite Verbesserung des wenigstens einen qualifizierenden Werts (z. B. Wahrscheinlichkeit oder Konfidenz) :

Es wird der wenigstens eine Bildgebungsparameter (z. B. Sequenz oder Kontrast) ausgewählt, der für möglichst viele klinischen Indikationen/Diagnosen in der Rangliste eine maximale Verbesserung des wenigstens einen qualifizierenden Werts verspricht (kumulierte maximale Änderung) .
"Wähle den wenigstens einen Bildgebungsparameter, der für alle potentiellen klinischen Indikationen, den höchsten Informationsgehalt hat (d.h. z. B. die Wahrscheinlichkeit und/oder die Konfidenz der Wahrscheinlichkeit am meisten verbessert)
→ Max(∑(Informationsgehalt für alle potentiellen klinischen Indikationen))

b. Fokus auf spezielle Verbesserung des wenigstens einen qualifizierenden Werts (einzelne maximale Änderung):

Es wird der wenigstens eine Bildgebungsparameter ausgewählt, der für eine bestimmte klinische Indikation/Diagnose eine maximale Verbesserung des wenigstens einen qualifizierenden Werts verspricht (z. B. weil durch eine bestimmte Sequenz eine bestimmte Diagnose sicher ausgeschlossen werden kann).
"Wähle den wenigstens einen Bildgebungsparameter, der für eine spezifische klinische Indikation, den höchsten Informationsgehalt hat (d.h. z. B. die Wahrscheinlichkeit und/oder die Konfidenz der Wahrscheinlichkeit am meisten verbessert)
→ Max(Informationsgehalt einer klinische Indikation)

b.1. Eine Variante von Kriterium b. ist, dass beispielsweise nur für die klinische Indikation, die aktuell die höchste oder niedrigste Wahrscheinlichkeit hat, die Konfidenz maximal verbessert werden soll und anhand dieses Kriteriums der nächste Bildgebungsparameter (z. B. Sequenz) ausgewählt wird.

b.2. Umgekehrt ist es möglich, dass die klinische Indikation, die am unwahrscheinlichsten ist, sicher ausgeschlossen werden soll und daher der nächste Bildgebungsparameter so ausgewählt wird, dass für diese klinische Indikation die Konfidenz maximal verbessert wird.

b.3. Es kann auch der Fokus auf eine bestimmte klinische Indikation gelegt werden, wenn bereits ein Verdacht zu einer bestimmten klinischen Indikation besteht. Dabei kann der wenigstens eine Bildgebungsparameter so ausgewählt werden, dass möglichst schnell eine hohe Konfidenz bei für die ausgewählte Indikation erreicht wird. Solch ein Vorgehen kann hilfreich sein, wenn man beispielsweise eilige Fälle wie Patienten mit Verdacht auf Schlaganfall untersucht.

c. Kombinationssuche: z. B. Höchster Informationsgehalt (Kriterium a.) bei möglichst geringer Zeit.

d. "Subsamplesuche": Fokus auf die Vermeidung bestimmter Bildgebungsparameter (Sequenzen) Dieses Vorgehen kann mit den bisherigen Kriterien a. bis c. kombiniert werden. Die Auswahl der Bildgebungsparameter kann so erfolgen, dass bestimmte Bildgebungsparameter (z. B. mit bestimmten Bildgebungseigenschaften (z. B. Zeitdauer, "Atemanhalten", Kontrastmittel usw.)) vermieden werden und trotzdem eine möglichst maximale Änderung des wenigstens einen qualifizierenden Werts (z. B. Wahrscheinlichkeit oder Konfidenz) erreicht wird. Ein solches Vorgehen ist hilfreich, wenn beispielsweise möglichst auf Kontrastmittel, "Atemanhalten" oder laute Sequenzen (z. B. Diffusion) verzichtet werden soll. Der Auswahlalgorithmus (SA) kann mit diesem Kriterium helfen die Sequenzen so auszuwählen, dass beispielsweise eine genügende Konfidenz erreicht wird, ohne dass die Sequenzen belastend für den Patienten sind.

"Suche nur innerhalb der Bildgebungsparameter mit bestimmten Bildgebungseigenschaften (z. B. die kein Atemanhalten erfordern)"

Durch die Auswahl geeigneter Kriterien für den Auswahlalgorithmus (SA) kann der Benutzer (Radiologe/MTRA) das Bildgebungsverfahren gezielt in eine bestimmte Richtung steuern, sofern dies erwünscht bzw. notwendig ist.

Gemäß einer Ausgestaltung der vorliegenden Erfindung ist der mindestens eine qualifizierende Wert eine Wahrscheinlichkeit der klinischen Indikationen oder eine Konfidenz einer Wahrscheinlichkeit der klinischen Indikationen.

Der Auswahlalgorithmus (SA) wählt den wenigstens einen Bildgebungsparameter (z. B. Sequenz oder Kontrast) basierend auf den Wahrscheinlichkeiten ($P_{Indication}$) und/oder den Konfidenzen ($C_{Indication}$) für die klinischen Indikationen in der Rangliste aus. So kann beispielsweise die Wahrscheinlichkeit, dass ein Hirntumor vorliegt, und die Konfidenz dieser Wahrscheinlichkeit, die auf der Analyse der vorherigen Bilddaten durch den Erkennungsalgorithmus (DA) beruht, bei der gezielten Auswahl von MRT-Sequenzen durch den Auswahlalgorithmus (SA) berücksichtigt werden bzw. kann die Auswahl darauf beruhen.

Die Wahrscheinlichkeit von klinischen Indikationen ermöglicht eine gezielte Fokussierung der Auswahl der Bildgebungsparameter für die erneute Bildgebung, um nur die plausibelsten klinischen Indikationen/Diagnosen weiter zu verfolgen. Die Konfidenz dieser Wahrscheinlichkeit ermöglicht es, die weitere Bildgebung durch die Auswahl der Bildgebungsparameter so zu steuern, dass mit den resultierenden Bilddaten nur Diagnosen mit hoher Sicherheit von dem Benutzer (z. B. Radiologe oder MTRA) gestellt werden.

Gemäß einer Ausgestaltung der vorliegenden Erfindung basiert in Schritt b) das Erstellen der Rangliste auf den Bilddaten aus allen vorherigen Iterationen.

Der Erkennungsalgorithmus (DA) berücksichtigt beim Erstellen der Rangliste potentieller klinischer Indikationen nicht nur die Bilddaten, die aktuell an das Auswahlmodul übertragen wurden, sondern auch vorherige Bilddaten aus vorherigen Iterationen des Bildgebungsverfahrens. Es können auch Bilddaten, die zu einem früheren Zeitpunkt, beispielsweise bei einem vorherigen Tumor-Staging, erstellt wurden, von dem Erkennungsalgorithmus (DA) berücksichtigt werden.

Insbesondere ein Erkennungsalgorithmus umfassend ein NN kann mit zusätzlichen Bilddaten des gleichen Patienten genauere Aussagen (z. B. höhere Konfidenz der Wahrscheinlichkeiten der potentiellen klinischen Indikationen) treffen und so eine verlässlichere Rangliste erstellen.

Gemäß einer Ausgestaltung der vorliegenden Erfindung ist der wenigstens eine Bildgebungsparameter eine Magnetresonanztomo-graphie-(MRT-)Sequenz.

Gemäß einer Ausgestaltung der vorliegenden Erfindung ist das Bildgebungsverfahren ein MRT-Verfahren.

Gemäß einer Ausgestaltung der vorliegenden Erfindung ist die Bildgebungsvorrichtung ein MRT-Gerät.

Insbesondere bei MRT-Geräten bzw. bei der MRT-Verfahren können durch die vielen verschiedenen MRT-Sequenzen, die viele verschiedene Kontraste, Schichtdicken ("Slice-Thickness"), Pulsmuster usw. umfassen, viele verschiedene Bilddaten erstellt werden, die jeweils für andere klinische Indikationen bzw. Diagnosen besonders geeignet sind. Durch das automatische Auswählen der MRT-Sequenzen kann somit bei MRT-Geräten/Verfahren eine besonders hohe Zeitersparnis und besonders große Steigerung der Sicherheit, mit der eine Diagnose durch den Benutzer gestellt wird, erreicht werden.

Gemäß einer Ausgestaltung der vorliegenden Erfindung wurde der primäre Bildgebungsparameter anhand einer primären Fragestellung (z. B. Informationen des überweisenden Arztes) ausgewählt.

Wenn eine primäre Fragestellung bekannt ist, beispielsweise "Verdacht auf Schlaganfall" oder bei einem geplanten Tumor-Staging, wird diese Information dazu verwendet den wenigstens einen primären Bildgebungsparameter (z. B. Sequenz oder Kontrast) so zu wählen, dass die resultierenden (primären) Bilddaten bereits möglichst gut zur Befundung der klinischen Fragestellung/Diagnose, die auf der primären Fragestellung beruht, möglich ist.

Durch die Berücksichtigung der primären Fragestellung kann die Zeitdauer und die Qualität der Bildgebung weiter gesteigert werden.

Gemäß einer Ausgestaltung der vorliegenden Erfindung werden, wenn das vordefinierte Abbruchkriterium erfüllt ist, zumindest die zuletzt generierten Bilddaten oder die zuletzt erstellte Rangliste ausgegeben.

Die Erfindung sowie das technische Umfeld werden nachfolgend anhand der Figuren näher erläutert. Es wird darauf hingewiesen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte und Merkmale zu extrahieren und mit anderen Bestandteilen und Erkenntnissen aus der vorliegenden Beschreibung und/oder Figuren zu kombinieren. Insbesondere wird darauf hingewiesen, dass die Figuren und insbesondere die dargestellten Größenverhältnisse nur schematisch sind. Gleiche Bezugszeichen bezeichnen gleiche Gegenstände, so dass ggf. Erläuterungen aus anderen Figuren ergänzend herangezogen werden können.

Figurenbeschreibung

Fig. 1    zeigt schematisch ein Ablaufdiagramm eines Verfahrens zum automatischen Auswählen von Bildgebungsparametern.

Fig. 2    zeigt schematisch ein Ablaufdiagramm eines Bildgebungsverfahrens einschließlich des Verfahrens zum automatischen Auswählen von Bildgebungsparametern aus Fig. 1.

Fig. 3    zeigt schematisch eine Bildgebungsvorrichtung umfassend eine Steuereinrichtung, die dazu eingerichtet ist, das Bildgebungsverfahren aus Fig. 2 durchzuführen.

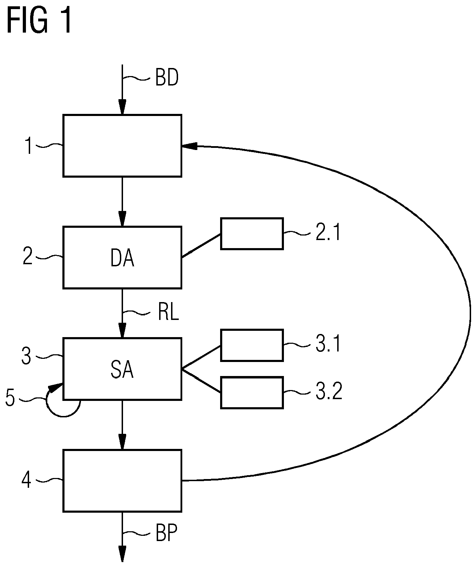

In Fig. 1 ist schematisch ein Ablaufdiagramm eines Verfahrens zum automatischen Auswählen von Bildgebungsparametern gemäß der vorliegenden Erfindung dargestellt. Indem ersten Schritt a) erfolgt ein Empfangen 1 von Bilddaten BD. Die Bilddaten können von einer Bildverarbeitungsvorrichtung wie beispielsweise einem Magnetresonanztomographie-(MRT-)Gerät, einem Computertomographie-(CT-)Gerät, Ultraschallgerät oder dergleichen erstellt worden sein. Die Bilddaten BD können dabei von einem Bildgebungsmodul der Bildverarbeitungsvorrichtung stammen und beispielsweise durch ein Auswahlmodul der Bildverarbeitungsvorrichtung empfangen werden. Basierend auf diesen empfangenen Bilddaten BD erfolgt in einem zweiten Schritt b) ein Erstellen 2 einer Rangliste RL mindestens eines qualifizierenden Werts für potentielle klinische Indikationen. Das Erstellen 2 kann zusätzlich auch auf Bilddaten BD aus vorherigen Iterationen oder auch auf Bilddaten aus anderen Bildgebungen basieren. Das Erstellen 2 der Rangliste RL erfolgt mittels eines Erkennungsalgorithmus (Detection Algorithm) DA. Der Erkennungsalgorithmus kann beispielsweise durch das Auswahlmodul ausgeführt werden. Nachdem die Rangliste RL erstellt wurde, erfolgt in einem dritten Schritt c) ein Auswählen 3 wenigstens eines Bildgebungsparameters BP. Der wenigstens eine Bildgebungsparameter BP ist geeignet, Bilddaten BD in einer nachfolgenden (erneuten) Bildgebung (z.B. MRT-Scan) zu erzeugen, die eine maximale Änderung der qualifizierenden Werte in der Rangliste RL ermöglichen. Das Auswählen 3 des wenigstens einen Bildgebungsparameters BP erfolgt mittels eines Auswahlalgorithmus (Selection Algorithm) SA. Der Auswahlalgorithmus kann beispielsweise durch das Auswahlmodul ausgeführt werden. Der ausgewählte wenigstens eine Bildgebungsparameter BP wird anschließend in einem vierten Schritt d) durch ein Senden 4 des ausgewählten Bildgebungsparameters BP, beispielsweise von dem Auswahlmodul an das Bildgebungsmodul übertragen. Der erste bis vierte Schritt werden so lange iterativ widerholt, bis ein Abbruchkriterium erfüllt ist.

Es erfolgt ein automatisches Auswählen von Bildgebungsparametern durch ein dynamisches Diagnoseausschlussprotokoll, das im Wesentlichen den Erkennungsalgorithmus DA und den Auswahlalgorithmus SA umfasst.

Der Erkennungsalgorithmus DA gibt eine Wahrscheinlichkeit $P_{In\text{-}dication}$ für das Vorhandensein einer bestimmten klinischen Indikation/Krankheit an. Gleichzeitig erfolgt über die Ermittlung einer Konfidenz $C_{Indication}$ der Wahrscheinlichkeit eine Aussage darüber, wie "sicher" diese ermittelte Wahrscheinlichkeit $P_{Indication}$ ist. Basis für diese Auswertung sind dabei die gerade empfangenen oder alle bisher empfangenen Bilddaten BD. Das heißt es erfolgt eine Bildanalyse, wobei beispielsweise in der 1. Iteration nur 1 Kontrast, in der 2. Iteration 2 Kontraste, in der 3. Iteration 3 Kontraste usw.

vorliegen, die zum Erstellen der Rangliste durch den Erkennungsalgorithmus DA analysiert werden. Dabei kann die Bildanalyse durch den Erkennungsalgorithmus mit Hilfe unterschiedlicher Verfahren durchgeführt werden. Es können neuronale Netzwerke (Neural Networks, NN) 2.1, insbesondere Deep NN (DNN) und Unterkategorien (z. B. Convolutional NN (CNN)) dazu eingesetzt werden. Möglich sind auch "Support Vector Machines" (SVMs) und "Random Forest Classifiers" (RFCs). Exemplarisch wird hier für ein NN 2.1 das Vorgehen zum Training und zur späteren Nutzung in einem MRT-Gerät (MR-Scanner) beschrieben:

1. Training & Test des NN (supervised learning):

Trainingsdaten:

- Bilddatensätze einzelner Sequenzen/Kontraste inklusive technischer Konfigurationsparameter (typischerweise im DICOM Header gespeichert)
- "Ground Truth": Information darüber, ob eine bestimmte klinische Indikation im Bild zu erkennen ist als boole'sche Variable (1 = klinische Indikation vorhanden; 0 = klinische Indikation nicht vorhanden). In Bilddaten von gesunden Patienten beispielsweise sind die booleschen Variablen für alle klinischen Indikationen = 0.
- Optional weitere Trainingsinformationen, wie beispielsweise welche Bilddaten zum gleichen Patient gehören.

Zum Training, Test und zur endgültigen Validierung können unterschiedliche Strategien verwendet werden.

2. Nutzung des NN 2.1 zur Generierung der qualifizierenden Werte, beispielsweise Wahrscheinlichkeiten für verschiedene klinische Indikationen $P_{Indication}$ und Konfidenzen dieser Wahrscheinlichkeiten $C_{Indication}$.

a. Laden der Bilddaten bisher durchgeführter Bildgebungen, beispielsweise MRT-Scans, im DICOM Format.
b. Anwendung des trainierten NN 2.1 welches die Wahrscheinlichkeiten $P_{Indication}$ und Konfidenzen $C_{Indication}$ zu den möglichen klinischen Indikationen (z. B. Hirn-Tumor) ermittelt.
c. Ausgabe:

- Die Wahrscheinlichkeit für das Vorhandensein einer bestimmten klinischen Indikation/Diagnose $P_{Indication}$.
- Die Konfidenz (Signifikanz)zu diesen Wahrscheinlichkeiten $C_{Indication}$.

Der auf den Erkennungsalgorithmus DA aufbauende Auswahlalgorithmus SA hat das Ziel, den nächsten wenigstens einen Bildgebungsparameter, hier die nächste Scansequenz (inkl. entsprechender Sequenzeinstellungen) für das MRT-Gerät, auszuwählen. Basis für die Entscheidung bzw. Auswahl ist dabei das Ergebnis, nämlich die Rangliste RL, des vorhergehenden Erkennungsalgorithmus DA (insbesondere die Wahrscheinlichkeit für das Vorhandensein einer bestimmten klinischen Indikation $P_{In-dication}$ und/oder die Konfidenz zu diesen Wahrscheinlichkeiten $C_{Indication}$). Es gibt unterschiedliche Möglichkeiten die Auswahl der nächsten Scansequenz vorzunehmen:
Zum einen "memory-based", wobei die Informationen zu den Bildgebungsparametern (Scansequenzen und Scaneinstellungen) und Eigenschaften der Bildgebungsparameter in einer Datentabelle 3.1 vorliegen. Die Datentabelle 3.1 enthält je Bildgebungsparameter drei Bereiche:

1. Scansequenz + Sequenzeinstellungen (z. B. FLAIR, "Slice Thickness" 2mm usw.)
2. Bildgebungseigenschaften (z. B. Zeitdauer, "Atemanhalten [ja/nein]" usw.)
3. Informationsgehalt für klinische Indikationen (z. B. boole'sch (ja = hat Informationswert; nein = hat keinen Informationswert) oder kontinuierlich (0 % = kein Informationswert bis 100 % = Krankheit kann alleine auf Basis dieser Scansequenz erkannt werden)).

Auf diese Datentabelle 3.1 können unterschiedliche Algorithmen zur Auswahl angewendet werden. Beispielsweises Vorgehen:

1. Durchsuchen der Bildgebungsparameter der Datentabelle und Auswahl der Bildgebungsparameter (Scansequenz und Sequenzeinstellung) für die die entsprechenden Suchkriterien gelten, zum Beispiel:

a. Fokus auf breite Verbesserung des wenigstens einen qualifizierenden Werts (z. B. Wahrscheinlichkeit oder Konfidenz) :

Es wird der wenigstens eine Bildgebungsparameter (z. B. Sequenz oder Kontrast) ausgewählt, der für möglichst viele klinischen Indikationen/Diagnosen in der Rangliste eine maximale Verbesserung des wenigstens einen qualifizierenden Werts verspricht (kumulierte maximale Änderung).

"Wähle den wenigstens einen Bildgebungsparameter, der für alle potentiellen klinischen Indikationen, den höchsten Informationsgehalt hat (d.h. z. B. die Wahrscheinlichkeit und/oder die Konfidenz der Wahrscheinlichkeit am meisten verbessert)

→ Max(∑(Informationsgehalt für alle potentiellen klinischen Indikationen))

b. Fokus auf spezielle Verbesserung des wenigstens einen qualifizierenden Werts (einzelne maximale Änderung):

Es wird der wenigstens eine Bildgebungsparameter ausgewählt, der für eine bestimmte klinische Indikation/Diagnose eine maximale Verbesserung des wenigstens einen qualifizierenden Werts verspricht (z. B. weil durch eine bestimmte Sequenz eine bestimmte Diagnose sicher ausgeschlossen werden kann).

"Wähle den wenigstens einen Bildgebungsparameter, der für eine spezifische klinische Indikation, den höchsten Informationsgehalt hat (d.h. z. B. die Wahrscheinlichkeit und/oder die Konfidenz der Wahrscheinlichkeit am meisten verbessert)

→ Max(Informationsgehalt einer klinische Indikation)

b.1. Eine Variante von Kriterium b. ist, dass beispielsweise nur für die klinische Indikation, die aktuell die höchste oder niedrigste Wahrscheinlichkeit hat, die Konfidenz maximal verbessert werden soll und anhand dieses Kriteriums der nächste Bildgebungsparameter (z. B. Sequenz) ausgewählt wird.

b.2. Umgekehrt ist es möglich, dass die klinische Indikation, die am unwahrscheinlichsten ist, sicher ausgeschlossen werden soll und daher der nächste Bildgebungsparameter so ausgewählt wird, dass für diese klinische Indikation die Konfidenz maximal verbessert wird.

b.3. Es kann auch der Fokus auf eine bestimmte klinische Indikation gelegt werden, wenn bereits ein Verdacht zu einer bestimmten klinischen Indikation besteht. Dabei kann der wenigstens eine Bildgebungsparameter so ausgewählt werden, dass möglichst schnell eine hohe Konfidenz bei für die ausgewählte Indikation erreicht wird. Solch ein Vorgehen kann hilfreich sein, wenn man beispielsweise eilige Fälle wie Patienten mit Verdacht auf Schlaganfall untersucht.

c. Kombinationssuche: z. B. Höchster Informationsgehalt (Kriterium a.) bei möglichst geringer Zeit.

d. "Subsamplesuche": Fokus auf die Vermeidung bestimmter Bildgebungsparameter (Sequenzen) Dieses Vorgehen kann mit den bisherigen Kriterien a. bis c. kombiniert werden. Die Auswahl der Bildgebungsparameter kann so erfolgen, dass bestimmte Bildgebungsparameter (z. B. mit bestimmten Bildgebungseigenschaften (z. B. Zeitdauer, "Atemanhalten", Kontrastmittel usw.)) vermieden werden und trotzdem eine möglichst maximale Änderung des wenigstens einen qualifizierenden Werts (z. B. Wahrscheinlichkeit oder Konfidenz) erreicht wird. Ein solches Vorgehen ist hilfreich, wenn beispielsweise möglichst auf Kontrastmittel, "Atemanhalten" oder laute Sequenzen (z. B. Diffusion) verzichtet werden soll. Der Auswahlalgorithmus (SA) kann mit diesem Kriterium helfen die Sequenzen so auszuwählen, dass beispielsweise eine genügende Konfidenz erreicht wird, ohne dass die Sequenzen belastend für den Patienten sind.

"Suche nur innerhalb der Bildgebungsparameter mit bestimmten Bildgebungseigenschaften (z. B. die kein Atemanhalten erfordern)"

2. Übergabe der ausgewählten Bildgebungsparameter an den MR-Scanner zur Konfiguration, sodass nun der Benutzer auf Basis der ausgewählten MRT-Sequenz den MRT-Scan initiieren kann.

Zum anderen kann das Auswählen der Bildgebungsparameter (Scansequenzen) auch zusätzlich oder alternativ "model-based" erfolgen. Die Informationen zum präferierten Bildgebungsparameter (Scansequenz) sind als Gewichte innerhalb eines mathematischen Modells (z. B. NN) 3.2 gespeichert. Das Training eines solchen NN kann beispielsweise auf Basis des Nutzerverhaltens/der Nutzungshäufigkeit erfolgen:

1. Training des Netzes (inkl. Test & Validierung):
Inputdaten zum Training:
klinische Indikationen und typische Bildgebungsparameter (Sequenzen & Sequenzeinstellungen) zur Diagnose (Protokolle).

2. Nutzung des trainierten NN zur Auswahl der nächsten Bildgebungsparameter (Scansequenz). Es können ähnliche Entscheidungskriterien wie oben für den "memory-based" Auswahlalgorithmus SA mit Datentabelle beschrieben verwendet werden:

    a. Auswahl der Scansequenz, die von den meisten Nutzern zur Diagnose einer spezifischen klinischen Fragestellung verwendet wurde:

max(Häufigkeit einer klinischen Indikation)

    b. Auswahl der Scansequenz, die von den meisten Nutzern im Durchschnitt zur Diagnose aller klinischen Fragestellungen verwendet wurde:

max(Durchschnitt(Häufigkeit aller klinischen Indikationen))

    c. und d. sind analog zu den Entscheidungskriterien die oben beschrieben wurden.

Es besteht die Möglichkeit zwischen dem Erkennungsalgorithmus DA und dem Auswahlalgorithmus SA eine Rückführungsschleife ("Feedback Loop") 5 einzufügen. Dabei wird nach der Auswahl der nächsten Scansequenz und der Berechnung der neuen Rangliste RL durch den Erkennungsalgorithmus DA diese Information an den Auswahlalgorithmus SA zurückgegeben. Die rückgeführte Information wird somit verwendet, um beispielsweise die Informationen in der Datentabelle 3.1 zu verbessern oder die Gewichte im Modell (NN) 3.2 anzupassen.

Die Iteration durch den ersten bis vierten Schritt wird abgebrochen, sobald das Abbruchkriterium erfüllt ist. Das Abbruchkriterium gibt beispielsweise eine mindeste Wahrscheinlichkeit ($T_{high}^{Indication}$) an mit der eine klinische Indikation vorliegen muss, damit diese bestätigt werden kann. Andersherum kann auch eine höchste Wahrscheinlichkeit als Abbruchkriterium ($T_{low}^{Indication}$) vorgegeben werden, bis zu der eine klinische Indikation als nicht vorliegend bestätigt werden kann. Zusätzlich oder alternativ kann eine mindeste Konfidenz als Abbruchkriterium ($T^{Confidence}$) angegeben werden, ab der eine Aussage über die Wahrscheinlichkeit einer klinischen Indikation als sicher angesehen werden kann.

Nachfolgend ist in einer ersten Tabelle beispielhaft eine mögliche Ausgabe einer Rangliste RL des Erkennungsalgorithmus DA basierend auf Bilddaten BD von einem Kopf eines Patienten angeführt.

| klinische Indikation | Wahrscheinlichkeit | Konfidenz |
| --- | --- | --- |
| Tumor | 5 % | 3 % |
| Multiple Sklerose | 2 % | 5 % |
| Alzheimer | 1 % | 2 % |
| Schlaganfall | 1 % | 6 % |

In der nachfolgenden zweiten Tabelle sind Beispielhaft die qualifizierenden Werte in einer Rangliste RL nach einer weiteren Iteration angegeben. Dabei ist hier von dem Auswahlalgorithmus SA beispielhaft eine MRT-Sequenz gewählt worden, mit der die klinische Indikation "Schlaganfall" sicher (hohe Konfidenz) ausgeschlossen (geringe Wahrscheinlichkeit) werden kann. Dies kann durch das zuvor beschriebene Kriterium b.1. erreicht werden.

| klinische Indikation | Wahrscheinlichkeit | Konfidenz |
| --- | --- | --- |
| Tumor | 5 % | 3 % |
| Multiple Sklerose | 1 % | 40 % |
| Alzheimer | 1 % | 60 % |
| Schlaganfall | 0.1 % | 98 % |

In der dritten Tabelle, die nachfolgend angegeben ist, ist die Rangliste RL aus der zweiten Tabelle in einer nachfolgenden Iteration basierend auf Bilddaten aktualisiert worden, die aus einer Bildgebung mit Bildgebungsparametern (MRT-Sequenz) stammen, die auch möglichst alle anderen klinischen Indikationen mit möglichst hoher Sicherheit ermittelbar machen. Dies ist durch das zuvor beschriebene Kriterium a. möglich.

| klinische Indikation | Wahrscheinlichkeit | Konfidenz |
| --- | --- | --- |
| Tumor | 10 % | 75 % |
| Multiple Sklerose | 1 % | 80 % |
| Alzheimer | 1 % | 60 % |
| Schlaganfall | 0.1 % | 99 % |

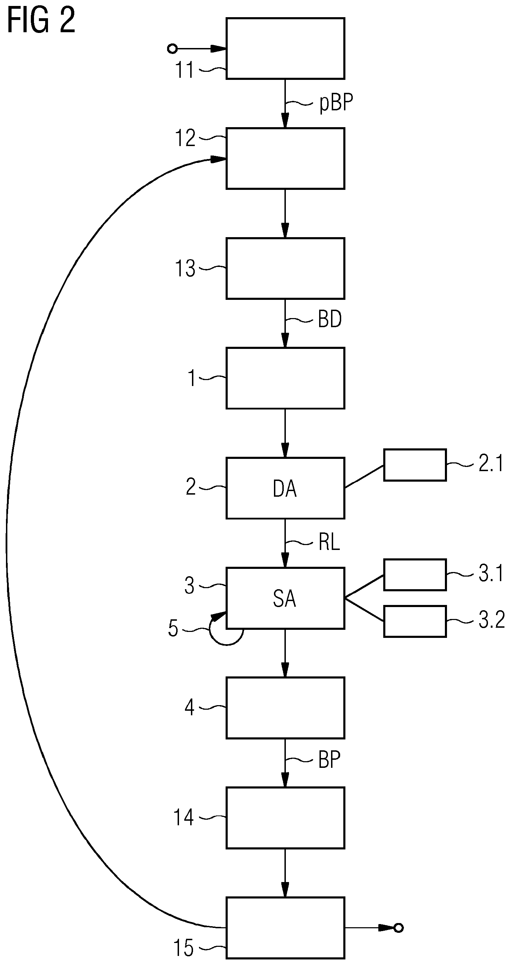

In Fig. 2 ist schematisch ein Ablaufdiagramm eines Bildgebungsverfahrens einschließlich des Verfahrens zum automatischen Auswählen von Bildgebungsparametern aus Fig. 1 dargestellt. Es werden nachfolgend nur die zusätzlichen Schritte des Bildgebungsverfahrens beschrieben. In einem ersten Bildgebungsschritt A) erfolgt ein Laden 11 wenigstens eines primären Bildgebungsparameters pBP, beispielsweise durch ein Bildgebungsmodul. Anschließend erfolgen in einem zweiten Bildgebungsschritt ein Durchführen 12 einer Bildgebung basierend auf dem geladenen Bildgebungsparameter pBP/BP und ein Generieren von Bilddaten BD (beispielsweise mit einer Bildgebungsvorrichtung wie einem MR-Scanner durch dessen Bildgebungsmodul). Darauf erfolgt in einem dritten Bildgebungsschritt ein Senden 13 der generierten Bilddaten BD (z.B. an ein Auswahlmodul des MR-Scanners durch dessen Bildgebungsmodul). Darauf folgen die vier Schritte das Verfahrens zum automatischen Auswählen von Bildgebungsparametern BP wie zuvor beschreiben. Nachdem wenigstens ein Bildgebungsparameter BP automatisch ausgewählt wurde, erfolgt in einem vierten Bildgebungsschritt ein Empfangen 14 des ausgewählten Bildgebungsparameters BP, der von dem Auswahlmodul des MR-Scanners stammen kann durch dessen Bildgebungsmodul. Schließlich erfolgt in einem fünften Bildgebungsschritt ein Laden 15 des empfangenen Bildgebungsparameters BP, beispielsweise durch das Bildgebungsmodul de MR-Scanners.

Der zweite bis dritte Bildgebungsschritt, der erste bis vierte Schritt des Verfahrens zum automatischen Auswählen von Bildgebungsparametern und der vierte bis fünfte Bildgebungsschritt erfolgen solange iterativ, bis das Abbruchkriterium wie zuvor beschrieben erfüllt ist.

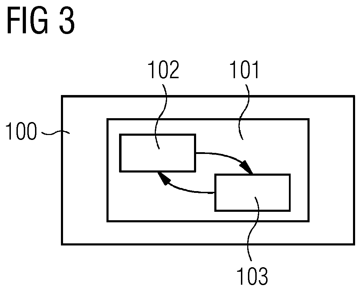

In Fig. 3 ist schematisch eine Bildgebungsvorrichtung umfassend eine Steuereinrichtung, die die eingerichtet ist, das Bildgebungsverfahren aus Fig. 2 durchzuführen, dargestellt. Die Bildgebungsvorrichtung 100, hier ein MRT-Gerät (MR-Scanner), umfasst eine Steuereinrichtung 101 mit einem Bildgebungsmodul 102 und einem Auswahlmodul 103. Das Bildgebungsmodul ist derart ausgestaltet und eingerichtet, dass es den ersten bis fünften Bildgebungsschritt ausführen kann. Das Auswahlmodul ist derart ausgestaltet und eingerichtet, dass es den ersten bis vierten Schritt des Verfahrens zum automatischen Auswählen von Bildgebungsparametern ausführen kann. Das Bildgebungsmodul 102 sendet dabei erzeugte Bilddaten BD an das Auswahlmodul 103 und das Auswahlmodul 103 sendet ausgewählte Bildgebungsparameter BP sowie optional ermittelte Ranglisten RL an das Bildgebungsmodul 102. Dabei kann die Steuereinrichtung 101 aus einer oder mehreren Datenverarbeitungseinrichtung, wie z. B. Mikrocontroller (pC), integrierte Schaltungen, anwendungsspezifische integrierte Schaltungen (Application-Specific Integrated Circuit, ASIC), anwendungsspezifisches Standardprodukte (Application-Specific Standard Products, ASSP), digitale Signalprozessoren (DSP), im Feld programmierbare (Logik-)Gatter-Anordnung (Field Programmable Gate Arrays, FPGA) und dergleichen aufgebaut sein. Das Bildgebungsmodul 102 und das Auswahlmodul können auf der bzw. den Datenverarbeitungseinrichtungen der Steuereinrichtung 101 ausgeführt sein oder jeweils eigene Datenverarbeitungseinrichtungen umfassen, die mit der Steuereinheit 101 kommunikativ verbunden sind.

Obwohl hier spezifische Ausführungsformen illustriert und beschrieben wurden, ist für den Fachmann ersichtlich, dass es eine Vielzahl von Alternativen und/oder gleichwertigen Implementierungen gibt. Es ist zu würdigen, dass die exemplarischen Ausgestaltungen oder Ausführungsformen nur Beispiele sind und nicht dazu gedacht sind, den Umfang, die Anwendbarkeit oder die Konfiguration in irgendeiner Weise einzuschränken. Vielmehr wird die vorstehende Zusammenfassung und detaillierte Beschreibung dem Fachmann hinreichende Anweisungen für die Umsetzung von mindestens einer bevorzugten Ausführungsform liefern, wobei es sich versteht, dass verschiedene Änderungen in der Funktion und Anordnung der Elemente, die in einer beispielhaften Ausgestaltung beschrieben werden, nicht von dem in den beigefügten Ansprüchen und ihren rechtlichen äquivalenten dargelegten Anwendungsbereich hinausführen. In der Regel ist diese Anmeldung dazu gedacht, alle Anpassungen oder Variationen der hier diskutierten spezifischen Ausführungsformen abzudecken.

In der vorstehenden ausführlichen Beschreibung wurden verschiedene Merkmale in einem oder mehreren Beispielen zusammengefasst, um die Offenbarung knapp zu halten. Es versteht sich, dass die obige Beschreibung illustrativ und nicht restriktiv sein soll. Sie soll alle Alternativen, Änderungen und äquivalente abdecken, die im Rahmen der Erfindung enthalten sein können. Viele andere Beispiele werden einem Fachmann bei dem Studium der obigen Offenbarung offensichtlich werden.

Um ein umfassendes Verständnis der Erfindung zu ermöglichen, wird eine spezifische Nomenklatur verwendet, die in der vorstehenden Offenbarung verwendet wurde. Es wird jedoch für einen Fachmann im Lichte der darin enthaltenen Spezifikation ersichtlich sein, dass die spezifischen Details nicht erforderlich sind, um die Erfindung zu anzuwenden. So werden die vorstehenden Beschreibungen spezieller Ausführungsformen der vorliegenden Erfindung zu Illustrations-und Beschreibungszwecken dargestellt. Sie sind nicht dazu gedacht, erschöpfend zu sein oder die Erfindung auf die oben offenbarten genauen Ausführungsformen zu beschränken; offensichtlich sind viele Modifikationen und Variationen im Hinblick auf die oben genannten Lehren möglich. Die Ausführungsformen wurden ausgewählt und beschrieben, um die Grundsätze der Erfindung und ihre praktischen Anwendungen am besten zu erklären und um somit anderen Fachkräften die Möglichkeit zu geben, die Erfindung und verschiedene Ausführungsformen mit verschiedenen Modifikationen, sowie es für die jeweilige Verwendung geeignet erscheint, am besten anzuwenden. In der gesamten Spezifikation werden die Begriffe "einschließlich" und "in welchem/welcher" als Äquivalente der jeweiligen Begriffe "umfassend" bzw. "worin" verwendet. Darüber hinaus werden die Begriffe "erster/erste/erstes", "zweiter/zweite/zweites", "dritter, dritte, drittes" usw. lediglich als Bezeichnung verwendet und sind nicht dazu gedacht, numerische Anforderungen an die Objekte zu stellen oder eine bestimmte Rangfolge vorzugeben. Im Zusammenhang mit der vorliegenden Beschreibung und den Ansprüchen ist die Verbindung "oder" als Aufnahme ("und/oder") zu verstehen und nicht exklusiv ("entweder... oder").

**Patentansprüche**

1. Verfahren zum automatischen Auswählen von Bildgebungsparametern (BP), umfassend die iterativen Schritte:

   a) Empfangen (1) von Bilddaten (BD);
   b) Erstellen (2) einer Rangliste (RL) mindestens eines qualifizierenden Werts für potentielle klinische Indikationen basierend auf zumindest den Bilddaten (BD) aus dem vorherigen Schritt mittels eines Erkennungsalgorithmus (DA);
   c) Auswählen (3) wenigstens eines Bildgebungsparameters (BP), der geeignet ist Bilddaten (BD) zu erzeugen, die eine maximale Änderung der qualifizierenden Werte in der Rangliste (RL) ermöglichen, mittels eines Auswahlalgorithmus (SA); und
   d) Senden (4) des ausgewählten Bildgebungsparameters (BP) ;

   wobei die Schritte a) bis d) wiederholt werden, bis ein vordefiniertes Abbruchkriterium erfüllt ist.

2. Verfahren gemäß Anspruch 1, wobei in Schritt c) das Auswählen (3) des wenigstens einen Bildgebungsparameters (BP) durch den Auswahlalgorithmus (SA) anhand einer Datentabelle (3.1) oder anhand eines Modells (3.2) erfolgt.

3. Verfahren gemäß Anspruch 1 oder 2, wobei das Abbruchkriterium erfüllt ist, wenn ein qualifizierender Wert wenigstens einer der klinischen Indikationen in der Rangliste (RL) ein vorgegebenes Grenzwertkriterium erfüllt oder alle möglichen Bildgebungsparameter (BP) durchlaufen sind.

4. Verfahren gemäß einem der vorherigen Ansprüche, wobei in Schritt c) die maximale Änderung der qualifizierenden Werte in der Rangliste (RL) auf einer maximalen Änderung eines der qualifizierenden Werte einer einzelnen klinischen Indikation in der Rangliste (RL) oder auf einer kumulierten maximalen Änderung qualifizierender Werte mehrerer klinischer Indikationen in der Rangliste (RL) beruht.

5. Verfahren gemäß einem der vorherigen Ansprüche, wobei der mindestens eine qualifizierende Wert eine Wahrscheinlichkeit der klinischen Indikationen oder eine Konfidenz einer Wahrscheinlichkeit der klinischen Indikationen ist.

6. Verfahren gemäß einem der vorherigen Ansprüche, wobei in Schritt b) das Erstellen (2) der Rangliste (RL) auf den Bilddaten (BD) aus allen vorherigen Iterationen basiert.

7. Verfahren gemäß einem der vorherigen Ansprüche, wobei der wenigstens eine Bildgebungsparameter (BP) eine Magnetresonanztomographie-, MRT-, Sequenz ist.

8. Bildgebungsverfahren einschließlich eines Verfahrens zum automatischen Auswählen von Bildgebungsparametern (BP) gemäß Anspruch 1, umfassend die Schritte:

   A) Laden (11) wenigstens eines primären Bildgebungsparameters (pBP);
   B) Durchführen (12) einer Bildgebung basierend auf dem geladenen Bildgebungsparameter (pBP, BP) und Generieren von Bilddaten (BD);
   C) Senden (13) der generierten Bilddaten (BD);

   a) Empfangen (1) der Bilddaten (BD);
   b) Erstellen (2) einer Rangliste (RL) mindestens eines qualifizierenden Werts für potentielle klinische Indikationen basierend auf zumindest den Bilddaten (BD) aus dem vorherigen Schritt mittels eines Erkennungsalgorithmus (DA);
   c) Auswählen (3) wenigstens eines Bildgebungsparameters (BP), der geeignet ist Bilddaten (BD) zu erzeugen, die eine maximale Änderung der qualifizierenden Werte in der Rangliste (RL) ermöglichen, mittels eines Auswahlalgorithmus (SA);

d) Senden (4) des ausgewählten Bildgebungsparameters (BP) ;

D) Empfangen (14) des ausgewählten Bildgebungsparameters (BP);
E) Laden (15) des empfangenen Bildgebungsparameters (BP) ;

wobei die Schritte B) bis C), a) bis d) und D) bis E) widerholt werden, bis ein vordefiniertes Abbruchkriterium erfüllt ist.

9. Bildgebungsverfahren gemäß Anspruch 7, einschließlich eines Verfahrens gemäß einem der Ansprüche 2 bis 6.

10. Bildgebungsverfahren gemäß Anspruch 7 oder 8, wobei der primäre Bildgebungsparameter (pBP) anhand einer primären Fragestellung ausgewählt wurde.

11. Bildgebungsverfahren gemäß einem der Ansprüche 7 bis 9, wobei, wenn das vordefinierte Abbruchkriterium erfüllt ist, zumindest die zuletzt generierten Bilddaten(BD) oder die zuletzt erstellte Rangliste (RL) ausgegeben werden.

12. Bildgebungsverfahren gemäß einem der Ansprüche 8 bis 10, einschließlich eines Verfahrens gemäß Anspruch 6, wobei das Bildgebungsverfahren ein MRT-Verfahren ist.

13. Bildgebungsvorrichtung (100), umfassend eine Steuereinrichtung (101) mit einem Bildgebungsmodul (102) und einem Auswahlmodul (103), die eingerichtet ist, ein Bildgebungsverfahren gemäß einem der Ansprüche 7 bis 11 durchzuführen, wobei das Bildgebungsmodul (102) eingerichtet ist, die Schritte A) bis C) und D) bis E) auszuführen, und wobei das Auswahlmodul (103) eingerichtet ist, die Schritte a) bis d) auszuführen.

14. Bildgebungsvorrichtung gemäß Anspruch 12, wobei die Steuereinrichtung eingerichtet ist, ein Bildgebungsverfahren gemäß Anspruch 11 durchzuführen und wobei die Bildgebungsvorrichtung ein MRT-Gerät ist.

# FIG 1

# FIG 2

FIG 3

Europäisches
Patentamt

European
Patent Office

Office européen
des brevets

# EUROPÄISCHER RECHERCHENBERICHT

Nummer der Anmeldung

EP 18 19 6783

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2009/118614 A1 (SENDAI TOMONARI [JP]) 7. Mai 2009 (2009-05-07) | 1-9, 11-14 | INV. G16H30/20 |
| Y | * Abstrakt, Par. 69, 85-87 * | 10 | |
| Y | US 2015/080733 A1 (YAMAMOTO HIROAKI [JP]) 19. März 2015 (2015-03-19) | 10 | |
| A | * Par. 92-93 * | 1-9, 11-14 | |
| A | US 2004/254439 A1 (FOWKES KENNETH M [US] ET AL) 16. Dezember 2004 (2004-12-16) * Par. 64, 67, 68 * | 1-14 | |
| A | Unknown: "Patient-centered CT imaging: New methods for patient-specific optimization", , 17. Dezember 2015 (2015-12-17), XP055528950, Gefunden im Internet: URL:http://incenter.medical.philips.com/doclib/enc/fetch/2000/4504/577242/577249/586938/587315/iPatient_WP_HR.pdf?nodeid=13207522&vernum=-2 [gefunden am 2018-11-30] * Seite 2, Par. 4-6 * | 1-14 | |

RECHERCHIERTE SACHGEBIETE (IPC)

G16H

Der vorliegende Recherchenbericht wurde für alle Patentansprüche erstellt

| Recherchenort | Abschlußdatum der Recherche | Prüfer |
|---|---|---|
| München | 25. März 2019 | Bankwitz, Robert |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 19 6783

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-03-2019

| Im Recherchenbericht angeführtes Patentdokument | Datum der Veröffentlichung | Mitglied(er) der Patentfamilie | Datum der Veröffentlichung |
|---|---|---|---|
| US 2009118614 A1 | 07-05-2009 | JP 5052123 B2<br>JP 2008161283 A<br>US 2009118614 A1 | 17-10-2012<br>17-07-2008<br>07-05-2009 |
| US 2015080733 A1 | 19-03-2015 | JP 5902558 B2<br>JP 2013244203 A<br>US 2015080733 A1<br>WO 2013176246 A1 | 13-04-2016<br>09-12-2013<br>19-03-2015<br>28-11-2013 |
| US 2004254439 A1 | 16-12-2004 | KEINE | |

EPO FORM P0461

Für nähere Einzelheiten zu diesem Anhang : siehe Amtsblatt des Europäischen Patentamts, Nr.12/82